Europäisches Patentamt

European Patent Office

Office européen des brevets

(11) Publication number: **0 409 242 A1**

# EUROPEAN PATENT APPLICATION

(21) Application number: 90113869.3

(51) Int. Cl.⁵: **C12P 21/08**, //A61K39/395

(22) Date of filing: **19.07.90**

The applicant has subsequently filed a sequence listing and declared, that it includes no new matter.

(30) Priority: **21.07.89 JP 187478/89**

(43) Date of publication of application:
**23.01.91 Bulletin 91/04**

(84) Designated Contracting States:
**DE FR GB**

(71) Applicant: **The Calpis Food Industry Co., Ltd.**
**20-3, 2-chome Ebisu-Nishi**
**Shibuya-ku Tokyo(JP)**

(72) Inventor: **Ikuta, Kazuyoshi**
**2-3, Nishi 9-chome, Minami 16-jo, Chuo-ku**
**Sapporo-shi, Hokkaido(JP)**
Inventor: **Ohki, Kohji**
**Ra Bahn No. 101, 9-chome, Shinkawa 4-jo**
**Kita-ku, Sapporo-shi, Hokkaido(JP)**
Inventor: **Ohmura, Kazutaka**
**3-3-14, Kitakashiwa**
**Kashiwa-shi, Chiba-ken(JP)**
Inventor: **Kato, Shiro**
**4-23-7 Fujishirodai**
**Suita-shi, Osaka-fu(JP)**

(74) Representative: **Bockhorni, Josef, Dipl.-Ing. et**
**al**
**Herrmann-Trentepohl, Kirschner, Grosse,**
**Bockhorni & Partner Forstenrieder Allee 59**
**59**
**D-8000 München 71(DE)**

(54) Monoclonal antibody against human CD4 peptide.

(57) The invention provides monoclonal antibodies reactable with a peptide consisting of an amino acid chain of 70th to 132nd amino acids, with a peptide consisting of an amino acid chain of 68th to 94th amino acids and with a peptide consisting of an amino acid chain of 86th to 104th amino acids each as counted from the N-terminal of a CD4 molecule.

EP 0 409 242 A1

# MONOCLONAL ANTIBODY AGAINST HUMAN CD4 PEPTIDE

## BACKGROUND OF THE INVENTION

This invention relates to a monoclonal antibody produced by using as an immunogen a fragmentary peptide of the human CD4 protein, that is a peptide consisting of an amino acid chain of 70th to 132nd amino acids as counted from the N-terminal of the CD4 molecule, referred to hereinafter as human CD4 peptide (70 - 132). The human CD4 protein is known as a receptor which is specifically bound with the envelope glycoprotein gp 120 of HIV (human immunodeficiency virus) causing the AIDS (acquired human immunodeficiency syndromes) and which causes T lymphatic cells to be infected by HIV (Dalgleish et al., Nature 312 , 763 - 767 (1984), Klatzmann et al., Nature 312 , 767 - 768 (1984), Mc. Dougal et al., Sciona 231 , 382 - 385 (1986), Sodroski et al., Nature 322 , 470 - 474 (1986) and Laski et al., Cell 50 , 975 - 985 (1987)). The human CD4 peptide (70-132) is also believed to be a region specifically bound with gp 120.

The human CD4 protein is a membrane protein traversing the cell membrane of the T lymphatic cell. It has been shown that the CD4 molecule is an HIV receptor protein and that a soluble CD4 molecule, that is a soluble molecule formed by producing only the outer membrane region of the CD4 molecule, competitively inhibits the binding between the CD4 and the HIV envelope glycoprotein gp 120 to exhibit an anti-HIV activity.

Thus, it has been recognized important to take note of the mechanism of infection with the CD4 molecule for the prevention and therapy of AIDS.

There are known monoclonal antibodies OKT 4A, B, C, D, E or F, Leu 3a or MT 151, which are prepared by immunizing a mouse with the whole human CD4 molecule as an immunogen. Of these, OKT 4A exhibits a strong HIV infection inhibiting activity and hence researches have been conducted especially on this antibody. However, the human CD4 peptides (11 - 40), (18 - 51), (18 - 69) or (26 - 47), which are the epitopes for this antibody, were not seen to exhibit an HIV infection inhibiting ativity (Jameson et al., Science 240 , 1335 - 1339 (1988)). Thus, it may be hardly contemplated that the HIV infection inhibiting action proper to OKT 14A results from the direct blocking of the binding region of the CD4 mlecule with gp 120 and the true binding region of the CD4 molecule with gp 120 has not been determined precisely. It has been desired to identify the true binding region in the CD4 molecule with gp 120 and to produce the corresponding monoclonal antibody.

## SUMMARY OF THE INVENTION

It is a principal object of the present inention to provide a monoclonal antibody that can be bound with a specific fragmentary peptide of the human CD4 molecule to inhibit the infection by HIV.

It is another object of the present invention to provide a monoclonal antibody necessary for analyses of the infection mechanism by HIV.

It is a further object of the present invention to provide a monoclonal antibody which may be utilized as an anti-HIV drug and a raw material for vaccine such as Fab fractions.

The above and other objects of the present invention will become apparent from the following description.

In accordance with the present invention, there is provided a monoclonal antibody reactable with a peptide consisting of an amino acid chain of 70th to 132nd amino acids as counted from the N-terminal of a CD4 molecule.

In accordance with the present invention, there is also provided a monoclonal antibody reactable with a peptide consisting of an amino acid chain of 68th to 94th amino acids as counted from the N-terminal of a CD4 molecule.

In accordance with the present invention, there is also provided a monoclonal antibody reactable with a peptide consisting of an amino acid chain of 86th to 104th amino acids as counted from the N-terminal of a CD4 molecule.

## DESCRIPTION OF THE PREFERRED EMBODIMENTS

The present invention will be explained in more detail hereinbelow.

When a monoclonal antibody is utilized for analyzing the binding region in the human CD4 molecule with an HIV protein gp 120, it may be contemplated that, since the antibody has a larger molecule size (about 150 kd) as compared with the size of the CD4 molecule (about 55 kd), the binding properties of the antibody with gp 120 may be affected by steric hindrance due to the difference in size, thus possibly resulting in mistaken judgment. The present inventors prepared 16 different partial peptides of the human CD4 molecule and on the basis of the competitive HIV inhibiting activity of each of the human CD4 peptides the inventors have conduted investigations into the possible binding regions in the CD4 molecule with gp 120. It may be contemplated that, since the size of these partial CD4 peptides is about one-seventh of the size of the CD4 molecule, steric hindrance may be minimized and hence the C4 partial peptide exhibiting an HIV infection inhibiting activity represents the true binding region of the human CD4 molecule with gp 120.

As a result of these investigations, it has been demonstrated that the human CD4 peptide (70 - 132), human CD4 peptide (68 - 94) and human CD4 peptide (86 - 104) exhibit an HIV infection inhibiting activity. Consequently, these partial peptides were synthesized chemically and inoculated to an animal as an immunogen to fuse the lymphocytes and myeloma cells of the animal to produce three strains of hybridoma cells. It has been demonstrated that these hybridoma cell strains produce an anti-CD4 peptide (70 - 132) antibody, an anti-CD4 peptide (68 - 94) antibody and an anti-CD4 peptide (86 - 104) antibody. More specifically, hybridoma CP-9 (FERM P-10864), deposited in the Fermentation Research Institute under International Deposit No. BP -3011, produces an antibody bindable with the CD4 peptide (70 - 132), hybridoma CP - 1 (FERM P-10862), deposited in the Fermentation Research Institute under International Deposit No. BP - 3009, produces an antibody bindable with the CD4 peptide (70 - 132) and CD4 peptide (68 - 94), and hybridoma CP - 2 (FERM P-10863), deposited in the Fermentation Research Institute under International Deposit No. BP -3010, produces an antibody bindable with the CD4 peptide (70 - 132) and CD4 peptide (86 - 104).

The present invention will be explained further with reference to the human CD4 peptide (70 - 132) according to an embodiment of the prsesent invention.

For preparing the monoclonal antibody of the present invention, the human CD4 peptide (70 - 132), which is to be the antigen, is prepared. According to the present embodiment, this peptide composed of 63 residues is prepared by chemical synthesis.

The above partial peptide is prepared with high purity by solid phase synthesis, employing an Fmoc amino acid as proposed by Sheppard et al., J. Chem. Soc., Chem. Comm., 165 - 166 (1985). The amino acid sequence is determined in accordance with the Maddon's Report (Maddon et al., Cell, 47 , 333 - 348 (1986)).

An Fmoc amino acid pentafluorophenyl (Pfp) ester corresponding to the C-terminal of the partial peptide is attached to a p-alkoxybenzyl alcohol resin in dimethylformamide in the presence of 4-dimethylamino pyridine. Then, another Fmoc amino acid Pfp ester to be attached next is attached by condensation reaction to the firstly mentioned Fmoc amino acid Pfp ester.

It is noted that threonine and serine are introduced by using 1-oxo-2-hydroxy-dihydro-benzotriazine (DEBT) ester. After termination of the coupling reaction of the amino acids, the resin with the peptide attached thereto is treated with a 20%-solution of piperidine in dimethylformamide to remove the terminal Fmoc group. The resulting product is acted upon by TFA - thioanisole at room temperature in the presence of m-cresol for de-protection and recovery of the object partial peptide from the resin. The peptide containing arginine residues is treated with trimethyl silyl bromide (TMSBr) - thioanisole / TFA.

The above described method of producing the CD4 fragmentary peptide is not limitative of the present invention. For example, it is also possible to produce the peptide by other chemical methods or to obtain a DNA corresponding to the fragmentary peptide and to insert the DNA into a suitable vector for reproduction in animal cells or in a culture medium to produce the object partial peptide. The in vitro immunization method with peripheral blood cells can also be advantageously used especially to obtain a human monoclonal antibody. A typical procedure to obtain an animal monoclonal antibody is as follows:

A mammal, preferably a mouse or rat, is immunized with the produced antigen. If a mouse is used, it has preferably 5 to 10 weeks of age. The antigen is administered in an amount of 0.001 to 1 mg and preferably 50 to 200 μg per dosage per one animal after mixing thoroughly with an adjuvant, such as complete Freund's adjuvant, by hypodermal injection, intravenous injetion or intraperitoneal injection. Subsequently, the antigen is administered 1 to 5 times at an interval of one to three weeks by hypodermal, intravenous or intraperitoneal injection after mixing thoroughly with an adjuvant, such as incomplete Freund's adjuvant.

Lymphocytes are sampled from the thus immunized animal in two to four days after the final immunization. Spleen, lymphoid nodules or peripheral blood is used for preparing the lymphocytes. These lymphocytes are fused with myeloma cells.

EP 0 409 242 A1

As the myeloma cells, those free of specific enzymes, such as, for example, mouse myeloma P3-X63-Ag8-U1 (P3-U1), P3-X63-Ag8-653(X63-653) or P3-NS1-1-Ag4-1 (NS-1) or rat myeloma 210-RCY3-Ag123, are preferred. Cell fusion may be performed in accordance with the Koehler and Milstein's method (nature 256 , 495 - 497 (1975)).

After the termination of the cell fusion, hybridomas are selected by using a selective culture medium, such as a HAT (hypoxanthine-aminopterin-thymidine) culture medium.

The hybridoma producing an antibody against the human CD4 peptide is selected by the enzyme immunoassay as later described and cloning by the limiting dilution method is repeated two or three times to produce the hybridoma cells producing the monoclonal antibody.

For investigating into which of the 63 amino acid residues of the human CD4 peptide (70 - 132) are recognized by the produced hybridomas, the human CD4 peptide (70 - 132) is roughly divided into three regions and the hybridomas are classified in accordance with the reactivity of the hybridomas with these regions. Specifically, on retrieval by enzyme immunoassay using the peptide (68 - 94), the peptide (86 - 104) and the peptide (105 - 120) as the antigens, there are obtained, besides the clonal strain reactable with the peptide (70 - 132) (hybridoma CP-9 deposited as FERM P-10864, BP-3011), the clonal strain reactable with the peptide (68 - 94) (hybridoma CP-1 deposited as FERM P-10862, BP-3009) and the clonal strain reactable with the peptide (86 - 104) (hybridoma CP-2, deposited as FERM P-10863, BP-3010). No clonal strain reactable with the peptide (105 - 120) was detected. The monoclonal antibody of the present invention may recognize at least three different epitopes, which is convenient in retrieving the binding site of the CD4 molecule with gp 120.

The object monoclonal antibody may be obtained by inoculating the monoclonal antibody producing hybridoma cell strains thus produced in the abdominal cavity of the animal of the same genus and collecting the abdominal dropsy after multiplication, or by cultivating the cell strains in a cultivator.

The produced antibody may be used after purification as the occasion may demand. Purification may be performed by any known methods usually employed for protein, such as, for example, ammonium sulfate fractionation, ion exchanging, gel filtration or affinity chromatography.

The monoclonal antibody obtained by the present invention is capable of reacting directly with the CD4 molecule existing in the CD4 (+) cell membrane, such as the cell membrane of the MOLT-4 or MT-4, as has been demonstrated by checking with the use of western blotting. About $1 \times 10^7$ of the above cells are collected and homogenized in a PBS (phosphate-buffered saline) containing 0.1 % of a surfactant, such as trade name "Triton X-100" or "NP 40" available from SIGMA CHEMICAL COMPANY and IWAI KAGAKU YAKUHIN CO., LTD., respectively, to produce $1 \times 10^5$ g of a supernatant on centrifugation for 60 minutes. This supernatant is concentrated by salting out by ammonium sulfate. This crude membrane fraction is separated by the SDS-10% polyacrylamido electrophoresis in accordance with Laemmli's method (Nature, 227 , 680 (1970)). Western blotting is then performed in accordance with the Towbin et al.'s method (Proc. Natl. Acad. Sci. USA, 76 , 4350 - 4354 (1979)). On coloration with a secondary antibody bound with peroxidase, a band with specific coloration is noticed at a zone corresponding to the molecular weight of 55,000 on the CD4 molecule, this band being also reactable with the anti-CD4 monoclonal antibody or OKT 4. It has been ascertained from this fact that the monoclonal antibody produced by using the CD4 peptide as the immunogen is also reactable with the CD4 protein.

Meanwhile, the monoclonal antibody obtained in accordance with the present invention is bindable with the CD4 molecule, while recognizing the specific binding site with HIV gp 120. On application to an infection model system between the HIV sensitive cell and HIV, it may be seen that the antibody exhibits an HIV infection inhibiting activity with an inhibit percentage of up to 75%.

EXAMPLES OF THE INVENTION

(1) Preparation of Immunized Mouse Spleen Cell

A BALB/c female mouse five weeks of age, supplied from Charles River Japan Inc., was immunized by intraperitoneal injection of 200 μg of the human CD4 peptide (70 - 132) simultaneously with complete Freund's adjuvant. Two weeks later, the animal was additionally immunized by intraperitoneal injection of 500 μg of the same peptide dissolved in a phosphate-buffered saline (PBS). The spleen was taken out after three days and disintegrated, suspended and washed in an RPMI-1640 culture medium to produce spleen cells for fusion.

4

## (2) Preparation of Mouse Myeloma Cells

The mouse myeloma P3-X63-Ag8-U1 was cultivated in a DMEM culture medium containing 10% of FCS and cells in the logarithmic growth phase were collected so as to be used as the parent strain for fusion.

## (3) Cell Fusion

$2 \times 10^8$ of the spleen cells and $2 \times 10^7$ of the mouse myeloma cells, obtained in (1) and (2) above, were mixed together thoroughly. The mixture was centrifuged and the produced supernatant was discarded to produce a pellet, which then was disintegrated thoroughly. 1 ml of a 50%-polyethylene glycol 1500, manufactured by Boehringer Mannheim Inc., was added gradually to the disintegrated mass over 1 to 2 minutes to effect cell fusion. Then, 10 ml of a DMEM culture medium was added dropwise over about ten minutes under agitation to complete the fusion. 1 ml of FCS was added to the reaction system to terminate the reaction of fusion.

The supernatant polyethylene glycol solution was removed by centrifugation. The produced pellet was washed with the DMEM culture medium containing 10% of FCS and the supernatant was removed by centrifugation. The pellet was suspended in a HAT culture medium (DMEM culture medium containing 100 $\mu$M of polyxanthine, 0.4 $\mu$M of aminopterin, 16 $\mu$M of thymidine, 0.03 % L-glutamine and 15% FCS) and poured onto a 96 well microtiter plate at a rate of 0.2 ml per well so that $5 \times 10^4$ cells are accommodated in each well. Cultivation was performed in a carbonic gas cultivator at 37°C and the HAT culture medium was renewed every other day at a rate of 0.1 ml per well. Cultivation and growth were continued for 7 to 10 days to produce hybridoma colonies.

## Enzyme Immunoassay

A 0.1 to 1 $\mu$g/ml solution of the human CD4 peptide dissolved in PBS was poured onto a 96 well microtiter plate for ELISA at a rate of 50 $\mu$l per well and allowed to stand at room temperature for 1 to 4 hours to allow the peptide to be adsorbed onto the bottom surface of each plate well. After washing three times with a phosphate buffer (PBS) with pH of 7.4 containing 0.15 M of sodium chloride, PBS containing 3% of casein sodium was charged into each well and allowed to stand for one hour at room temperature for coating the plate wells in their entirety with casein. After discarding the liquid in each well and washing with PBS, a supernatant liquid of the cultured hybridoma was added at a rate of 50 $\mu$l per well and allowed to stand at room temperature for 1 to 4 hours. After washing five times with PBS containing 0.05% Tween 20 available from Wako Pure Chemical Industries, Ltd. and washing twice with PBS, a solution of an anti-mouse IgG antibody bound with peroxidase manufactured by Nippon Bio-Rad Laboratories, diluted to a concentration of 1/4,000 with PBS, was poured at a rate of 50 $\mu$l per well, and allowed to stand at room temperature for one to two hours. After washing five times with 0.05% Tween 20-PBS and twice with PBS, a coloring liquid prepared by mixing a 50 mM sodium acetate buffer containing 10 mM orthophenylenediamine and 0.025% of BSA (pH 5.0) and 0.1% hydrogen peroxide at a ratio of 3 : 1 was added at a rate of 50 $\mu$l per well and allowed to stand at room temperature for to 30 minutes for reaction. 1N sulfuric acid containing 0.1% sodium sulfite ($Na_2SO_3$) was added to the reaction system at a rate of 150 $\mu$l per well. After termination of the reaction, measurement was performed at a wavelength of 492 nm. In retrieving the object hybridoma, it suffices to select hybridoma strains with the high value of measurement.

## (4) Hybrodoma Screening and Cloning

The supernatant of the cultured liquid in each well was taken and subjected to the above mentioned enzyme immunoassay. The human CD4 peptide (70 - 132) was used as the antigen. The hybridoma in each well in which the antibody was produced was cloned by the limiting dilution method to produce 11 clonal strains (CP1-11) producing the anti-human CD4 peptide (70 - 132) antibody.

## (5) Retrieval of the Epitope of the Monoclonal Antibody

Using each of the human CD4 peptides (68 - 94), (86 - 104) and (105 - 120) as the antigen, the supernatant of the culture medium in each well which reacted with the human CD4 peptide (70 - 132) was subjected as the specimen to the above mentioned enzyme immunoassay. A clonal strain reactable with the peptide (68 - 94) and two clonal strains reactable with the peptide (86 - 104) were produced (Table 1).

### (6) Purification of Monoclonal Antibody

Each 50 ml of the 11 hybridoma strains produced in (5) above was cultivated in a culture medium free of serum ASF103 manufactured by Ajinomoto Co. Ltd. The cultured products with the cell concentration of $1 \times 10^6$/ml were centrifuged and the produced supernatants were subjected to the protein A -agarose manufactured by Nippon Bio-Rad Laboratories or to the LCA lectin-cephalose manufactured by Pharmacia Inc. The supernatants subjected to the protein A-agarose manufactured by Nippon Bio-Rad Laboratories were eluted by a citric acid buffer solution with pH of 4.0, while those subjected to the LCA lectin-cephalose were eluted by PBS containing 10% methyl-$\alpha$-D-mannopyranoside. The eluted products were dialyzed by PBS to produce purified monoclonal antibodies (CP-11).

### (7) Reactivity of Monoclonal Antibody with CD4 Molecule

The reactivity of the monoclonal antibodies produced in (6) above was checked by using the western blotting method.

### Checking of the Reactivity with CD4 molecule by the Western Blotting Method

$1 \times 10^7$ of the MOLT-4 cells (CD4 + ) were suspended in 3 ml of PBS containing 0.1% Triton X-100 available from SIGMA CHEMICAL COMPANY and homogenized by a Potter type homogenizer. The resulting mass was centrifuged at $1 \times 10^5$ g for one hour and the supernatant was taken as the crude membrane fraction. After addition of ammonium sulfate, the precipitated 80% saturated ammonium sulfate fraction was dialyzed and freeze-dried so as to be used as the specimen for electrophoresis. After separation by electrophoresis with 10% - polyacrylamide gel containing 0.1% SDS, the protein was electrically transferred from the polyacrylamide gel to a nitrocellulose paper in a 25 mM trishydroxymethylaminomethane - 192 mM glycine buffer solution of pH 8.3 containing 20% methanol. The nitrocellulose paper was immersed in 1% casein - PBS at room temperature for one hour for coating. The protein was reacted with each monoclonal antibody at 4°C overnight, washed and reacted with a solution, diluted to a concentration of 1/400, of the anti-mouse IgG (H + L) marked with peroxidase, manufactured by Nippon Bio-Rad Laboratories, at room temperature for about two hours. After washing, the protein was reacted with a peroxidase substrate liquid containing 0.5 mg/ml of 4-chloro-1-naphtol, manufactured by Seikagaku Kogyo Co., Ltd. and 0.02% of hydrogen peroxide at room temperature for about 30 minutes to check the protein band where coloration was noticed.

The 11 kinds of the monoclonal antibodies produced as in (6) above were specifically reacted only with the protein corresponding to the molecular weight 55,000 of the human CD4 molecule.

Table 1

| Retrieval of Epitopes for Monoclonal Antibodies | | | | |
|---|---|---|---|---|
| Monoclonal Antibodies | Human CD4 Peptides | | | |
| | (70-132) | (68-94) | (86-104) | (105-120) |
| CP-1 | (+) | (+) | | |
| CP-2 | (+) | | (+) | |
| CP-3 | (+) | | | |
| CP-4 | (+) | | | |
| CP-5 | (+) | | | |
| CP-6 | (+) | | | |
| CP-7 | (+) | | | |
| CP-8 | (+) | | | |
| CP-9 | (+) | | | |
| CP-10 | (+) | | | |
| CP-11 | (+) | | (+) | |
| (+) indicates positive reactivity | | | | |

The primary amino acid sequences of the human CD4 peptides in accordance with the present invention are as follows:

(1) Peptide (70 - 132)
Pro-Leu-Ile-   Ile-Lys-Asn-Leu-Lys-Ile-Glu-Asp-Ser-Asp-Thr-Tyr-Ile-Cys-Glu-Val-Glu-Asp-Gln-Lys-Glu-Glu-Val-Gln-Leu-Leu-Val-Phe-Gly-Leu-Thr-Ala-Asn-Ser-Asp-Thr-His-Leu-Leu-Gln-Gly-Gln-Ser-Leu-Thr-   Leu-Thr-Leu-Glu-Ser-Pro-Pro-Gly-Ser-Ser-Pro-Ser-Val-Gln-Cys

(2) Peptide (68 - 94)
Asn-Phe-Pro-Leu-Ile-Ile-Lys-Asn-Leu-Lys-Ile-Glu-Asp-Ser-Asp-Thr-Tyr-Ile-Cys-Glu-Val-Glu-Asp-Gln-Lys-Glu-Glu

(3) Peptide (86 -104)
Cys-Glu-Val-Glu-Asp-Gln-Lys-Glu-Glu-Val-Gln-Leu-Leu-Val-Phe-Gly-Leu-Thr-Ala

(4) Peptide (105 - 120)
Asn-Ser-Asp-Thr-His-Leu-Leu-Gln-Gly-Gln-Ser-Leu-Thr-Leu-Thr-Leu

## Claims

1. A monoclonal antibody reactable with a peptide consisting of an amino acid chain of 70th to 132nd amino acids as counted from the N-terminal of a CD4 molecule.

2. A monoclonal antibody reactable with a peptide consisting of an amino acid chain of 68th to 94th amino acids as counted from the N-terminal of a CD4 molecule.

3. A monoclonal antibody reactable with a peptide consisting of an amino acid chain of 86th to 104th amino acids as counted from the N-terminal of a CD4 molecule.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | WO-A-8801304 (THE TRUSTEES OF COLUMBIA UNIVERSITY IN THE CITY OF NEW YORK) * claims * | 1-3 | C12P21/08 // A61K39/395 |
| X | ARCH. VIROL. vol. 105, no. 1-2, 1989, Vienna, Austria pages 129 - 135; Y. HAYASHI et al.: "Inhibition of HIV-1 replication and syncytium formation by synthetic CD4 peptides" * abstract * | 1 | |
| A | SCIENCE vol. 241, 05 August 1988, Washington DC, USA pages 712 - 716; J. LIFSON et al.: "Synthetic CD4 peptide derivatives that inhibit HIV infection and cytopathicity" * the whole document * | 2 | |

TECHNICAL FIELDS SEARCHED (Int. Cl.5 )

C07K
C12N
C12P

The present search report has been drawn up for all claims

| Place of search | Date of completion of the search | Examiner |
|---|---|---|
| THE HAGUE | 05 OCTOBER 1990 | NOOIJ F.J.M. |